# EUROPEAN PATENT SPECIFICATION

(11) **EP 0 411 582 B1**
(45) Date of publication and mention of the grant of the patent: **20.09.1995**
(21) Application number: 90114708.2
(22) Date of filing: 31.07.1990
(51) Int. Cl.: C12P 1/04, A01N 63/02, C12N 1/20

(54) **Bacillus thuringiensis var. donegani and preparation or toxin obtained therefrom, endowed with insecticidal activity against coleoptera**
Bacillus thuringiensis var. donegani und seine Erzeugung oder Toxine davon, mit einer insektiziden Wirkung gegen Käfer
Bacillus thuringiensis var. donegani et sa préparation ou toxine obtenue de lui, dotés d'une activité insecticide contre les coléoptères

(30) Priority: 01.08.1989 IT 2141289
(43) Date of publication of application: 06.02.1991
(73) Proprietor: MINISTERO DELL' UNIVERSITA' E DELLA RICERCA SCIENTIFICA E TECNOLOGICA, 00196 Roma (IT)
(72) Inventor: Cidaria, Dante, Dr., I-28100 Novara (IT); Cappai, Andrea, Dr., I-30135 Venezia (IT); Vallesi, Adriana, Dr., I-28100 Novara (IT); Caprioli, Vincenzo, Dr., I-27028 S. Martino Siccomario, Pavia (IT); Pirali, Giorgio, Dr., I-21047 Saronno, Varese (IT)
(74) Representative: Weinhold, Peter, Dr.

(56) References cited:
- EP-A- 0 149 162
- EP-A- 0 202 739

## Description

The present invention relates to a variety of Bacillus thuringiensis, denominated "var. donegani" (Btd) NCIMB 40152, and to a preparation and toxin obtainable therefrom, endowed with insecticidal activity against Coleoptera.

It is well known that the sporogenous bacillus Bacillus thuringiensis (Bt) produces, during sporogenesis, parasporal crystals of proteinic nature which display an insecticidal activity against a large number of insects belonging to the orders of Lepidoptera, Diptera and Coleoptera. Said crystals, when ingested by an insect sensitive to their activity, cause irreversible damages to its intestinal mucosa, with the insect consequently stopping feeding and dying.

Many varieties of Bt are known, which differ from one another with respect to their biochemical and/or physiological parameters, and their specific/selective insecticidal activity. Some of these varieties are marketed as formulations for use in the control of insects.

The Bt varieties known heretofore are subdivided, on the basis of their specific/selective activity, into three pathotypes:

| Pathotype | Specific insecticidal activity | Main varieties |
|---|---|---|
| A | Lepidoptera | kurstaki |
| B | Diptera | israelensis |
| C | Coleoptera | tenebrionis; san diego |

As pathotype C, endowed with specific activity against Coleoptera, only two varieties are known in the art, i.e., var. tenebrionis, described in EP-A-149,162 and var. san diego, disclosed in EP-A-202,739.

Object of the instant invention is the provision of a Bt variety and an insecticidal preparation and toxin obtainable therefrom, which are endowed with a superior specific insecticidal activity against Coleoptera and the preparation of which is simpler, cheaper and more efficient.

A variety of Bacillus thuringiensis, pathotype C, denominated var. donegani (Btd.) NCIMB 40152, and the corresponding insecticidal preparation and toxin have now been found which, besides being more advantageous than the Bt's, pathotype C, known heretofore, thanks to its insecticidal activity of the covering type, its preparation and its application in the agrarian field, unexpectedly is endowed with a systemic insecticidal activity.

Therefore, one object of the present invention is Bacillus thuringiensis variety donegani NCIMB 40152 or a biologically pure culture thereof.

Another object of the instant invention is the crystalline, parasporal proteinic toxin obtainable from cultures of Bacillus thuringiensis variety donegani NCIMB 40152 after sporogenesis and successive lysis of the sporangium, as claimed in claim 2.

A further object of the instant invention is an insecticidal preparation containing, as active principle, the crystalline, parasporal proteinic toxin obtainable from cultures of Bacillus thuringiensis variety donegani NCIMB 40152 after sporogenesis and successive lysis of the sporangium, as claimed in claim 3.

Still another object of the present invention is an insecticidal preparation wherein the active principle comprises solid, lyophilized products obtainable from the fermentation and sporogensis of Btd NCIMB 40152, as claimed in claim 4.

A still further object of the instant invention is a method for controlling insects, in particular Coleoptera, which method comprises the treatment of plants or parts thereof, such as stems and leaves, or the ground on which they are grown, with an efficient amount of Btd NCIMB 40152, a preparation and/or toxin derived therefrom, as such or in the form of a suitable composition containing inert, solid or liquid carriers and, optionally, other additives, as claimed in claim 5.

Bacillus thuringiensis var. donegani, (Btd) NCIMB 40152 is endowed with a specific insecticidal activity against Coleoptera and, in particular, against important agronomical targets, such as leaf beetle Leptinotarsa decemlineata (Doriphora of potato) and therefore belongs to "pathotype C" according to the classification by A. Krieg et al., Z. Ang. Ent. 96 (1983) 500-508.

Btd NCIMB 40152 differs from the known varieties of Bt in that it shows different biochemical and physiological characteristics and, in particular, is different from the tenebrionis and san diego varieties also in that it produces a parasporal crystal with different morphological characteristics, as well as with a different behaviour from a physico-chemical and physiological point of view.

### Morphology and Biochemical Characteristics of Btd NCIMB 40152

Bacillus thuringiensis var. donegani (Btd) NCIMB 40152 was isolated from dead larvae (owing to natural causes) of Tenebrio molitor.

The isolation was accomplished on Nutrient Agar medium, rendered selective by means of the addition of 10 U/ml of Penicillin G. The contents of the insects were preliminarily treated at 80°C for 15 to 20 minutes, for the purpose of selectively isolating the sporogenous bacilli. A culture of this microorganism was submitted on May 31,1989, in compliance with the Budapest Treaty to the National Collection of Industrial Bacteria (c/o The National Collection of Industrial and Marine Bacteria Ltd., Torry Research Station, P.0. Box 31, 135 Abbey Road, Aberdeen AB 98 DG, Scotland, U.K.), where it was given the access number NCIMB 40152.

Btd NCIMB 40152 can be maintained on conventional media, e.g., on Nutrient Agar medium at 25 to 30°C, and can be propagated by means of techniques per se known to those skilled in the art.

The determination of the genus and of the species was carried out on the basis of the morphological and biochemical characteristics of the microorganism.

### Morphology

The isolated colonies of Btd NCIMB 40152 on Nutrient Agar medium are flat, with circular or irregular shape and an eroded or lobate edge; they are of whitish colour. The vegetative cell has the shape of a small rod, is Gram positive, has a diameter of from 1 to 2 µm, and a length of from 6 to 8 µm.

The growth temperature ranges from 25 to 35°C.

When the culture of the microorganism is treated in an autoclave at 121°C for 15 minutes, both the spores and the toxin become inactivated.

The position of the spore is central, or sub-terminal. The parasporal crystal, observed under the electron scanning microscope, has the shape of a square-base parallelepipedon with two pyramids with equal bases on the upper face and on the lower face, with dimensions of from 1 to 2 µm; therefore it is different from the parasporal crystals produced by Bt tenebrionis and san diego varieties which are flat and rhomboidal in both cases.

### Biochemical Characteristics

Btd NCIMB 40152, in its vegetative step, has the biochemical characteristics reported in Table 1.

**Table 1**

| Test | Response |
|---|---|
| Acidification of glucose | + |
| Acidification of mannitol | - |
| Acidification of mannose | - |
| Acidification of saccharose | + |
| Acidification of lactose | - |
| Acidification of arabinose | - |
| Acidification of xylose | - |
| Acidification of salicin | + |
| Hydrolysis of esculin | + |
| Hydrolysis of starch | + |
| Hydrolysis of gelatin | + |
| Production of gas from glucose | - |
| Arginine di-hydrolase | - |
| Lysine decarboxylase | - |
| Phenyl-alanine deaminase | - |
| Urease | - |
| Catalase | + |
| (Soy) lecithinase | + |
| Nitrate reductase | + |
| Voges-Proskauer reaction | + |
| Growth on chitin | - |
| Growth in NaCl at 7% | - |
| Growth at 50°C | - |
| Growth in 10 U/ml of Penicillin G | + |
| Growth in 10 µg/ml of chloramphenicol | - |
| Growth in 25 µg/ml of streptomycin | - |
| Growth in 30 µg/ml of nalidixic acid | - |
| Growth in 50 µg/ml of lysozime | + |
| (as regards the test method used, see: "The Prokaryotes", Springer-Verlag, 1981; "Manual of Methods for General Bacteriology", The American Society for Microbiology, 1981.) | |

The difference between Btd 40152 and the varieties known from the prior art can also be inferred from a comparison of the biochemical characteristics of Btd 40152 to those of the known varieties of Bt, as reported in following Table 2.

**Table 2**

| [According to A. Krieg in "The Prokaryotes", Springer-Verlag, 1981, page 1750; A. Krieg, J. Appl. Ent. 104 (1987), 417-424] | | | | | | | | |
|---|---|---|---|---|---|---|---|---|
| Variety | MAN | SAC | SAL | AMI | CHT | URE | ARG | LCT |
| thuringiensis | + | + | + | + | V | - | + | |
| finitimus | - | + | + | - | + | - | - | |
| alesti | - | - | - | + | V | - | + | |
| kurstaki | - | - | + | + | V | + | + | |
| sotto | - | + | - | + | V | - | - | |
| dendrolimus | - | - | - | + | + | - | V | |
| keniae | - | - | + | + | V | + | + | |
| galleriae | - | - | + | + | V | + | + | |
| canadensis | - | + | + | + | + | - | + | |
| entomocidus | + | + | - | + | - | - | - | |
| aizawai | - | - | + | + | + | + | + | |
| morrisoni | - | + | - | + | V | - | - | |
| tolworti | - | + | + | + | V | - | + | |
| darmstadiensis | - | - | - | + | - | - | + | |
| toumanoffi | - | - | - | + | - | + | + | |
| thompsoni | - | + | + | + | V | + | + | |
| pakistani | - | + | (+) | + | (+) | - | + | |
| israelensis | + | - | - | + | - | - | | |
| wuhanensis | - | - | + | + | | - | | |
| tenebrionis | + | + | - | + | - | - | + | - |
| san diego | + | + | - | + | - | - | + | - |
| NCIMB 40152 | - | + | + | + | - | - | - | + |
| Footnotes to Table 2: MAN, SAC, SAL, acidification of mannose, saccharose and salicin; AMI, hydrolysis of starch; CHT, growth on chitin; URE, urease; ARG, arginine di-hydrolase; LCT, lecithinase; + = positive response; - = negative response; (+) = partially positive response; V = variable response. | | | | | | | | |

### Characteristics Relating to Culture and Methods of separation of the Active Principle

Bacillus thuringiensis var. donegani NCIMB 40152 can be cultivated on common liquid media, such as, e.g., Nutrient broth, at 25-30°C, by means of techniques known in the art.

At the end of the fermentation and sporogenesis process, the biomass can be separated by centrifuging the fermentation broth.

The thus separated biomass can subsequently be subjected to evaporation or lyophilization, so as to obtain a solid product in powder form which is endowed with insecticidal activity.

The insecticidal activity of Btd NCIMB 40152 is substantially due to a parasporal, crystalline toxin of proteinic nature, which - in contrast to the toxins produced by Bt var. tenebrionis and var. san diego - is soluble in water at neutral pH. Therefore, said toxin can be separated by resuspending the biomass separated by centrifugation from the fermentation broth after the sporogenesis in water, at a neutral pH, and keeping the thus obtained suspension, with stirring, at room temperature for 1 to 12 hours.

After the subsequent centrifugation or filtration of the aqueous suspension, an aqueous solution free of spores and fermentation residues can be recovered, which substantially contains the proteinic toxin, which can be recovered from its solution by means of well-known techniques such as, e.g., lyophilization.

The determination of the molecular weight of the toxin by SDS-electrophoresis on polyacrylamide gel, according to the method of Laemmli (Nature, 227, 680, 1970) evidences two main groups of bands:
* a first group of three bands, with a molecular weight within the range of from 64,000 to 72,000;
* a second group of two bands, with a molecular weight within the range of from 34,000 to 36,000.

### Biological Activity

Btd NCIMB 40152 and the preparation and toxin obtained from it, show an insecticidal activity, of the covering type as well as of the systemic type, against Coleoptera, such as, e.g., Leptinotarsa decemlineata.

In contrast thereto, no toxic effects were observed as regards some Lepidoptera such as, e.g., spodoptera littoralis and Ephestia kuehniella, as well as with the dipteran Aedes aegypti.

The action of the active principle on the larvae of Coleoptera is the same as observed in the past in the case of preparations on the basis of Bt "pathotype A": a few hours after the treatment, the larvae stop feeding and die after 24 to 48 hours. The death takes place owing to the effect of the toxin on the intestinal epithelium of the insect, which undergoes irreversible lesions.

Btd NCIMB 40152, the lyophilized solid products obtained from its fermentation and sporogenesis, as well as its parasporal proteinic toxin, as such or in the form of a suitable composition, hence is useful for controlling Coleoptera which infest agrarian cultivations.

For this purpose, the use of the isolated toxin is preferred.

Compared to the toxins obtainable from the known varieties of Bt and, in particular, of "pathotype C" varieties tenebrionis and san diego, the toxin derived from Btd NCIMB 40152 according to the present invention shows numerous advantages, at the financial-industrial level, with respect to its recovery, application and insecticidal activity.

Such advantages are, e.g.:
* the separation of the toxin from the culture medium is simpler and easier, thanks to its solubility in water, and, consequently, said toxin can be obtained in concentrated form free of spores, cells and fermentation residues;
* the concentrated aqueous solutions of the toxin - which, in contrast to the suspensions or dispersions of the toxins known from the prior art, do not settle - can be stored and transported more easily;
* the toxin can be applied, as solution, more easily and effectively than the analogous toxins which have to be applied as solids in dispersed form, owing to their incompatibility with the organic solvents conventionally used in formulations.

Furthermore, it has surprisingly been found that the toxin derived from Btd NCIMB 40152, besides being endowed with an insecticidal activity of the covering type, also shows - in contrast to the toxins produced by other varieties of Bt - a systemic insecticidal activity.

Said systemic activity, which is reflected by the migration of the active principle to plant parts even very far remote from the point of application, ensures a wider protection of the plant and makes it possible to use smaller amounts of active principle. In particular, the toxin according to the present invention moves via the root apparatus of the plants.

Furthermore, the toxin is effective for longer periods of time since, by penetrating the vascular system of the plant, it is better protected from the external agents which tend to cleave it or to wash it away.

The systemic activity of the toxin is even more advantageous when plants are treated which are in their growth step and, hence, offer to the insect young sprouts grown after the treatment.

Furthermore, the systemic activity endows the toxin with a higher efficiency in preventive treatments and decreases the need for repeated treatments during the plant growth cycle.

Bacillus thuringiensis var. donegani NCIMB 40152, the product obtained from its fermentation and sporogenesis, as well as its parasporal toxin, can be formulated, e.g., as concentrated liquids and wettable powders, by using liquid or solid inert vehicles and, optionally, auxiliary formulation agents and/or additives, such as wetting agents, adhesion promoters, surfactants, U.V. stabilizers and still other additives normally used in insecticidal formulations.

For practical use in agriculture, doses of from 0.05 to 4 kg/hectare, and preferably from 0.2 to 2 kg/hectare, of the toxin derived from Btd NCIMB 40152 are particularly suitable.

The following non-limitative examples are to illustrate the present invention.

### EXAMPLE 1

500 ml Erlenmeyer flasks containing 100 ml of a medium consisting of Tryptone® Soy Broth (OXOID) were inoculated with 1 ml of a 16-hour-old culture of Bacillus thuringiensis NCIMB 40152 in Nutrient Broth as the growth medium.

The Erlenmeyer flasks were kept, with orbital stirring, at temperatures of from 25 to 35°C. The sporogenesis was monitored under the microscope. At the end of the fermentation the biomass was centrifuged off, resuspended in water at pH 7 and kept, with stirring, for 3 hours at room temperature. The solution obtained after separation by centrifuging off the spores and the fermentation residues was lyophilized and used for the determination of biological activity or the preparation of the formulations.

### EXAMPLE 2

The determination of the insecticidal activity of the lyophilized preparations obtained according to example 1, substantially composed of the parasporal proteinic toxin, was carried out by using said preparation in the form of an aqueous solution at various concentrations.

The details of carrying out the activity tests, according to the insect used, are given in the following:

### LEPTINOTARSA DECEMLINEATA

### Covering activity:

Second-stage larvae were fed with young potato plants, treated by dipping them into an aqueous solution of the lyophilized toxin of example 1.

The environmental conditions during the observation were as follows:
* temperature 25 ± 1°C
* relative humidity 60 ± 2%

The final determination of the death rate was carried out 48 hours after the infection; the results are reported in Table 3.

### Systemic activity

Second-stage larvae of the insect were fed with young potato plants, kept for 12 hours and 24 hours, respectively in a hydroponic culture containing the lyophilized product prepared in example 1, dissolved at a concentration of 200 ppm.

After keeping the larvae for 48 hours in a conditioned environment, at 25°C and a R.H. of 60%, a death rate of 100% was determined in both tests.

### TRIBOLIUM CONFUSUM, TENEBRIO MOLITOR, EPHESTIA KUEHNIELLA

Third stage larvae were grown under controlled conditions, at a temperature of 25 ± 1°C and a relative humidity of 65 ± 2%, in white meal to which the lyophilized product of example 1 had previously been added.

The death rate was determined 14 days after the infestation; the results are reported in Table 3.

### AEDES AEGYPTI

### Activity

Third-stage larvae were placed in distilled water containing the lyophilized preparation of example 1. The larvae were kept under controlled conditions, at 25 ± 1°C and a relative humidity of 60%, and were fed daily. The death rate, reported in Table 3, was measured 5 days after the treatment.

### SPODOPTERA LITTORALIS

### Covering activity:

Second-stage larvae were fed with tobacco leaves, treated by dipping them into an aqueous solution of the lyophilized product of example 1.

The environmental conditions during the observation were as follows:
* temperature 25 ± 1°C
* relative humidity 60 ± 2%

The determination of the death rate was carried out 5 days after the infection; the results are reported in Table 3.

**TABLE 3**

| Order | Species | Dose (ppm) | Death rate |
|---|---|---|---|
| Coleoptera | Leptinotarsa decemlineata | 100 | 100 % |
| | Tenebrio molitor | 1,000 | 100 % |
| | Tribolium confusum | 1,000 | 100 % |
| Lepidoptera | Ephestia kuehniella | 1,000 | 0 % |
| | Spodoptera littoralis | 500 | 0 % |
| Diptera | Aedes aegypti | 500 | 0 % |

## Claims (Claims for the following Contracting State(s): AT, BE, CH, DE, DK, FR, GB, LI, NL, SE)

1. Bacillus thuringiensis variety donegani NCIMB 40152 or a biologically pure culture thereof.

2. The crystalline, parasporal proteinic toxin obtainable from cultures of Bacillus thuringiensis variety donegani NCIMB 40152 after sporogenesis and successive lysis of the sporangium, said toxin being soluble in water at neutral pH and being endowed with a systemic insecticidal activity against Coleoptera.

3. An insecticidal preparation, containing as active principle the crystalline, parasporal proteinic toxin according to claim 2.

4. An insecticidal preparation, wherein the active principle comprises the lyophilized solid products endowed with systemic insecticidal activity against Coleoptera obtainable from the fermentation and sporogenesis of Bacillus thuringiensis variety donegani NCIMB 40152.

5. Method for controlling insects, which comprises treating plants or parts thereof or the soil on which they are grown with Bacillus thuringiensis variety donegani NCIMB 40152, a toxin according to claim 2 and/or a preparation according to claims 3 or 4 derived from it, as such or in the form of a suitable composition which contains inert, solid or liquid vehicles and, optionally other additives.

## Claims (Claims for the following Contracting State(s): ES)

1. Process for controlling insects, which comprises treating plants or parts thereof or the soil on which they are grown with Bacillus thuringiensis variety donegani NCIMB 40152, a crystalline, parasporal proteinic toxin obtainable from cultures of Bacillus thuringiensis variety donegani NCIBM 40152 after sporogenesis and successive lysis of the sporangium, said toxin being soluble in water at neutral pH and being endowed with a systemic insecticidal activity against Coleoptera, and/or an insecticidal preparation containing as active principle said crystalline, parasporal proteinic toxin and/or an insecticidal preparation, wherein the active principle comprises the lyophilized solid products endowed with systemic insecticidal activity against Coleoptera obtainable from the fermentation and sporogenesis of Bacillus thuringiensis variety donegani NCIMB 40152, as such or in the form of a suitable composition containing inert, solid or liquid vehicles and, optionally, other additives.

## Patentansprüche (Patentansprüche für folgende(n) Vertragsstaat(en): AT, BE, CH, DE, DK, FR, GB, LI, NL, SE)

1. Bacillus thuringiensis Varietät donegani NCIMB 40152 oder eine biologisch reine Kultur desselben.

2. Kristallines, parasporales proteinisches Toxin, erhältlich aus Kulturen des Bacillus thuringiensis Varietät donegani NCIMB 40152 nach Sporogenese und anschließender Lyse des Sporangiums, wobei das Toxin löslich ist in Wasser bei neutralem pH und ausgestattet ist mit einer systemischen insektiziden Aktivität gegen Coleoptera.

3. Insektizide Zubereitung, die als aktives Prinzip das kristalline, parasporale proteinische Toxin gemäß Anspruch 2 enthält.

4. Insektizide Zubereitung, in der das aktive Prinzip die lyophilisierten festen, mit einer systemischen insektiziden Wirkung gegen Coleoptera ausgestatteten Produkte, die erhältlich sind durch Fermentierung und Sporogenese des Bacillus thuringiensis Varietät donegani NCIMB 40152, umfaßt.

5. Verfahren zum Bekämpfen von Insekten, welches umfaßt: Behandeln von Pflanzen oder deren Teilen oder der Erde, auf der sie wachsen, mit Bacillus thuringiensis Varietät donegani NCIMB 40152, einem Toxin gemäß Anspruch 2 und/oder einer Zubereitung gemäß Anspruch 3 oder 4, von diesem abgeleitet, als solche oder in Form einer geeigneten Zusammensetzung, die inerte, feste oder flüssige Vehikel und gegebenenfalls andere Zusätze enthält.

## Patentansprüche (Patentansprüche für folgende(n) Vertragsstaat(en): ES)

1. Verfahren zum Bekämpfen von Insekten, welches umfaßt: Behandeln von Pflanzen oder deren Teilen oder der Erde, auf der sie wachsen, mit Bacillus thuringiensis Varietät donegani NCIMB 40152, einem kristallinen, parasporalen proteinischen Toxin, erhältlich aus Kulturen des Bacillus thuringiensis Varietät donegani NCIMB 40152 nach Sporogenese und anschließender Lysis des Sporangiums, wobei das Toxin löslich ist in Wasser bei neutralem pH und ausgestattet ist mit einer systemischen insektiziden Aktivität gegen Coleoptera, und/oder einer insektiziden Zubereitung, die als aktives Prinzip das kristalline, parasporale proteinische Toxin enthält, und/oder einer insektiziden Zubereitung, in der das aktive Prinzip die lyophilisierten festen, mit einer systemischen insektiziden Wirkung gegen Coleoptera ausgestatteten Produkte umfaßt, die erhältlich sind durch Fermentierung und Sporogenese des Bacillus thuringiensis Varietät donegani NCIMB 40152, als solche oder in Form einer geeigneten Zusammensetzung, die inerte, feste oder flüssige Vehikel und gegebenenfalls andere Zusätze enthält.

## Revendications (Revendications pour l'(les) Etat(s) contractant(s) suivant(s): AT, BE, CH, DE, DK, FR, GB, LI, NL, SE)

1. *Bacillus thuringiensis variété donegani* NCIMB 40152 ou une culture biologiquement pure de celui-ci.

2. Toxine cristalline parasporale de nature protéique que l'on peut obtenir à partir de cultures de *Bacillus thuringiensis variété donegani* NCIMB 40152 après sporogénèse et lyses successives du sporange, ladite toxine étant soluble dans l'eau à pH neutre et dotée d'une activité insecticide systémique contre les coléoptères.

3. Préparation insecticide conforme à la revendication 2, caractérisée en ce que son principe actif est la toxine cristalline parasporale de nature protéique

4. Préparation insecticide, dans laquelle le principe actif est constitué des produits lyophilisés solides, dotés d'une activité insecticide systémique contre les coléoptères, que l'on peut obtenir à partir de la fermentation et de la sporogénèse de *Bacillus thuringiensis variété donegani* NCIMB 40152.

5. Procédé pour limiter la prolifération des insectes, qui consiste à traiter les plantes ou une partie de celles-ci ou le sol sur lequel elles sont cultivées par une toxine conforme à la revendication 2 et/ou une préparation conforme aux revendications 3 ou 4 dérivées de *Bacillus thuringiensis variété donegani* NCIMB 40152, sous la forme d'une composition convenable qui contient dès transporteurs inertes, solides ou liquides et éventuellement d'autres additifs.

## Revendications (Revendications pour l'(les) Etat(s) contractant(s) suivant(s): ES)

1. Procédé pour limiter la prolifération des insectes, qui consiste à traiter les plantes ou une partie de celles-ci ou le sol sur lequel elles sont cultivées par *Bacillus thuringiensis variété donegani* NCIMB 40152, une toxine cristalline parasporale de nature protéique que l'on peut obtenir à partir de cultures de *Bacillus thuringiensis variété donegani* NCIMB 40152 après sporogénèse et lyses successives du sporange, ladite toxine étant soluble dans l'eau à pH neutre et dotée d'une activité insecticide systémique contre les coléoptères, et/ou une préparation insecticide dont le principe actif est ladite toxine cristalline parasporale de nature protéique et/ou une préparation insecticide dans laquelle le principe actif est constitué des produits lyophilisés solides, dotés d'une activité insecticide systémique contre les coléoptères, que l'on peut obtenir à partir de la fermentation et de la sporogénèse de *Bacillus thuringiensis variété donegani* NCIMB 40152, en l'état ou sous la forme d'une composition convenable qui contient des transporteurs inertes, solides ou liquides et éventuellement d'autres additifs.
